# EUROPEAN PATENT APPLICATION

(11) **EP 3 524 313 A1**
(43) Date of publication of application: **14.08.2019**
(21) Application number: 17865276.4
(22) Date of filing: 05.10.2017
(51) Int. Cl.: A61M 35/00, B05C 17/00, B65D 83/00

(54) **COATING TOOL AND LIQUID MEDICINE COATING TOOL**

(30) Priority: 28.10.2016 JP 2016211917
(71) Applicant: Nipro Corporation, Osaka-shi, Osaka 531-8510 (JP)
(72) Inventor: SAWADA, Hidenori, Osaka-shi Osaka 531-8510 (JP); FUKETA, Hiroshi, Osaka-shi Osaka 531-8510 (JP); KAWAMURA, Naohisa, Osaka-shi Osaka 531-8510 (JP)
(74) Representative: Prüfer & Partner mbB Patentanwälte · Rechtsanwälte
(86) International application number: PCT/JP2017/036253
(87) International publication number: WO 2018/079219

(57) **Abstract**

An applicator includes: a liquid medicine container (130) having an opening (124), and a brush member (160) arranged adjacently to the liquid medicine container (130) to cover the opening (124). The brush member is made up of a plurality of fibers (140) that are bundled together. The brush member (160) has a tubular shape in a root portion located adjacently to the liquid medicine container (130), tapers from the root portion toward a top end portion located opposite to the liquid medicine container (130), and has a wedge shape in the top end portion,

## Description

### TECHNICAL FIELD

The present invention relates to an applicator and a liquid drug applicator.

### BACKGROUND ART

Japanese Patent Laying-Open Nos. 2013-13634 (PTL 1) and 2008-169163 (PTL 2) are prior documents each disclosing a configuration of an applicator.

The applicator disclosed in PTL 1 includes a liquid medicine container and a columnar brush member. The columnar brush member is made up of synthetic fibers bundled together in a columnar shape, and is arranged at an opening of the liquid medicine container. The top end portion of the columnar brush member has a fan shape enlarging in the widthwise direction perpendicular to the longitudinal direction of the column axis. The thickness of the top end portion in the lengthwise direction perpendicular to the longitudinal direction of the column axis decreases toward the topmost end of the columnar brush member.

The applicator disclosed in PTL 2 includes a receptacle portion containing an externally applied composition, an opening allowing the receptacle to communicate with the outside, and an applicator portion made up of many brush fibers disposed to cover the opening. The opening is formed by a cylindrical pipe. The brush fibers cover the periphery of the opening.

### CITATION LIST

### PATENT LITERATURE

PTL 1: Japanese Patent Laying-Open No. 2013-13634
PTL 2: Japanese Patent Laying-Open No. 2008-169163

### SUMMARY OF INVENTION

### TECHNICAL PROBLEM

Regarding the applicator disclosed in PTL 1, the top end portion of the brush member enlarges in a fan shape, and the top end portion of the brush member is very soft. The top end portion of the brush member is therefore likely to spread irregularly, resulting in a difficulty for the top end portion of the brush member to reach a deep site between a nail and skin.

As for the applicator disclosed in PTL 2, the top end portion of the brush member is not shaped to taper toward the topmost end of the brush member, resulting in a difficulty for the top end portion of the brush member to reach a deep site between a nail and skin.

The present invention has been made in view of the above-described problems. An object of the present invention is to provide an applicator and a liquid drug applicator that are capable of delivering a liquid medicine to a site located deep in a narrow gap while alleviating stimulus that may be given when the liquid medicine is applied.

### SOLUTION TO PROBLEM

An applicator based on the present invention includes: a liquid medicine container having an opening; and a brush member arranged adjacently to the liquid medicine container to cover the opening, the brush member being made up of a plurality of fibers that are bundled together. The brush member has a tubular shape in a root portion located adjacently to the liquid medicine container, tapers from the root portion toward a top end portion located opposite to the liquid medicine container, and has a wedge shape in the top end portion.

In an embodiment of the present invention, the applicator further includes a tubular member in which the brush member is inserted from the top end portion, and which is secured to the liquid medicine container. A part of the top end portion of the brush member is exposed to an outside of the tubular member from one end portion, located opposite to the liquid medicine container, of the tubular member, The one end portion has an inner peripheral surface in an elliptical shape. The inner peripheral surface is in contact with an outer periphery of the brush member.

In an embodiment of the present invention, the brush member further includes an annular member having one main surface to which the plurality of fibers are bonded. The annular member is arranged adjacently to the liquid medicine container to allow an inner space of the annular member to continue into an inner space of the opening.

In an embodiment of the present invention, a dimension of a length of the part of the top end portion of the brush member is larger than a dimension of a width of a root of the part of the top end portion of the brush member.

In an embodiment of the present invention, respective lengths of the plurality of fibers are identical to each other.

A liquid drug applicator based on the present invention includes: an applicator according to any one of the foregoing; and a liquid drug contained in the liquid medicine container.

### ADVANTAGEOUS EFFECTS OF INVENTION

In accordance with the present invention, a liquid medicine can be delivered to a site located deep in a narrow space, while stimulus that may be given when the liquid medicine is applied is alleviated.

### BRIEF DESCRIPTION OF DRAWINGS

Fig. 1 is a perspective view showing an external appearance of an applicator according to an embodiment of the present invention.
Fig. 2 is a cross-sectional view of the applicator in Fig. 1 along line II-II as seen in the direction of arrows.
Fig. 3 is a cross-sectional view of the applicator in Fig. 1 along line III-III as seen in the direction of arrows.
Fig. 4 is a perspective view of the applicator according to an embodiment of the present invention, with its cap removed.
Fig. 5 is a cross-sectional view showing a shape of a container body included in a liquid medicine container of the applicator according to an embodiment of the present invention.
Fig. 6 is a cross-sectional view showing a shape of an outlet port included in the liquid medicine container of the applicator according to an embodiment of the present invention.
Fig. 7 is a plan view showing an external appearance of an annular member included in a brush member of the applicator according to an embodiment of the present invention.
Fig. 8 is a side view showing an external appearance of the brush member of the applicator according to an embodiment of the present invention.
Fig. 9 is a cross-sectional view of the brush member in Fig. 8 along line IX-IX as seen in the direction of arrows.
Fig. 10 is a cross-sectional view of the same cross section as Fig. 2, showing a shape of a tubular member of the applicator according to an embodiment of the present invention.
Fig. 11 is a cross-sectional view of the same cross section as Fig. 3, showing a shape of the tubular member of the applicator according to an embodiment of the present invention.
Fig. 12 is a plan view showing a shape of a part of a top end portion of a brush member of an applicator for Comparative Example 1.
Fig. 13 is a side view showing a shape of the part of the top end portion of the brush member of the applicator for Comparative Example 1.
Fig. 14 is a plan view showing a shape of a part of a top end portion of a brush member of an applicator for Comparative Example 2.
Fig. 15 is a cross-sectional view showing a state in which a top end portion of a brush member of an applicator is inserted in a narrow gap in a first experimental example.
Fig. 16 is a graph showing results of a second experimental example.

### DESCRIPTION OF EMBODIMENTS

An applicator according to an embodiment of the present invention is described hereinafter with reference to the drawings. In the description of the embodiment, the same or corresponding parts are denoted by the same reference characters, and a description thereof is not repeated.

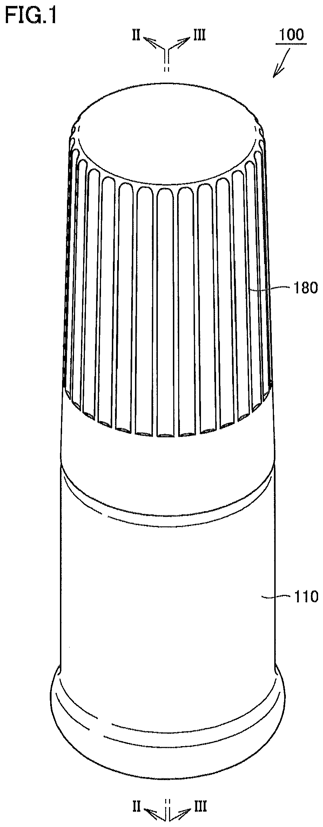

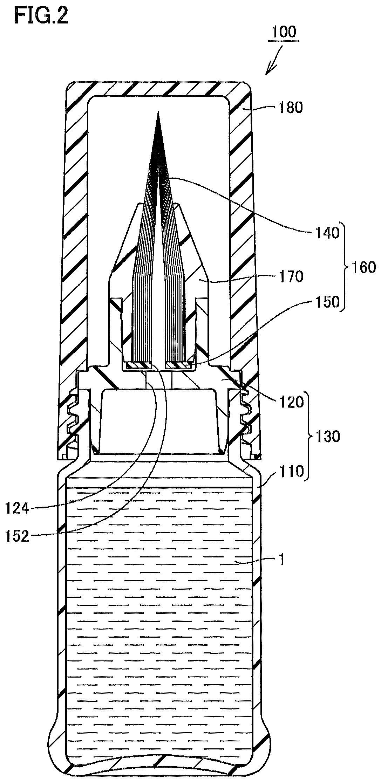

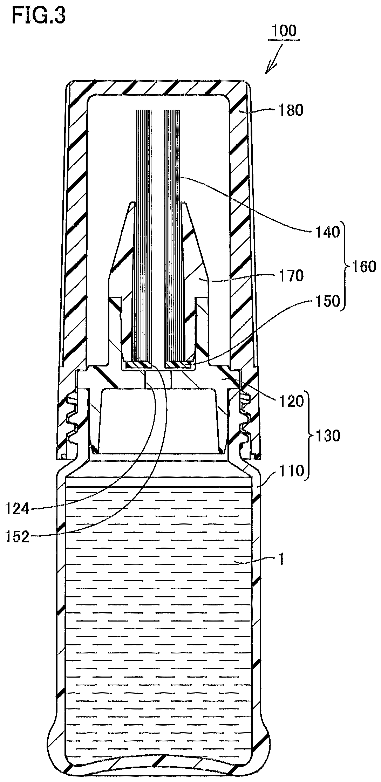

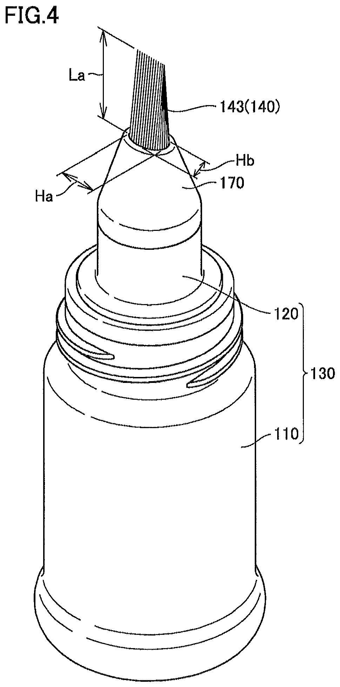

Fig. 1 is a perspective view showing an external appearance of an applicator according to an embodiment of the present invention. Fig. 2 is a cross-sectional view of the applicator in Fig. 1 along line II-II as seen in the direction of arrows. Fig. 3 is a cross-sectional view of the applicator in Fig. 1 along line III-III as seen in the direction of arrows. Fig. 4 is a perspective view of the applicator according to an embodiment of the present invention, with its cap removed.

As shown in Figs. 1 to 4, an applicator 100 according to an embodiment of the present invention includes a liquid medicine container 130 and a brush member 160. In the present embodiment, applicator 100 further includes a tubular member 170 and a cap 180.

As shown in Figs. 1 and 2, for a liquid drug applicator in which a liquid medicine contained in liquid medicine container 130 is a liquid drug 1, a liquid drug for onychomycosis treatment or a liquid drug for dermatophytosis treatment, for example, is used as liquid drug 1. The liquid drug for onychomycosis treatment may be a liquid drug containing efinaconazole, tavaborole, luliconazole, itraconazole, terbinafine, amorphin, ciclopirox, or the like. The liquid drug for dermatophytosis treatment may be a liquid drug containing miconazole, isoconazole, iconazole, clotrimazole, butenafine, lanoconazole, ketoconazole, econazole, oxiconazole, griseofulvin, tolnaftate, bifonazole, neticonazole, liranaftate, tolciclate, or the like. The liquid medicine contained in liquid medicine container 130 is not limited to above-indicated liquid drugs 1, and any of other liquid medicines may be contained.

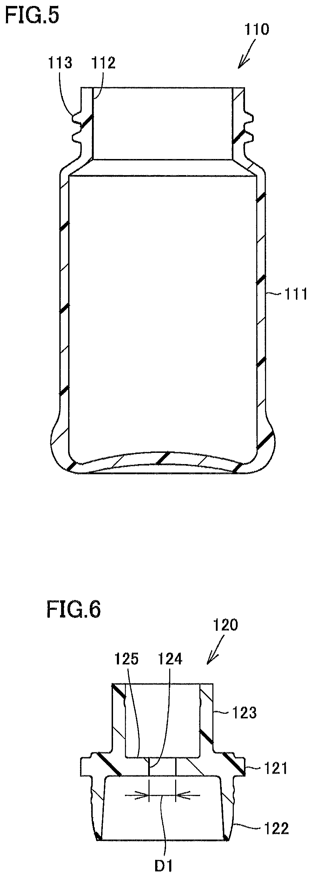

Liquid medicine container 130 includes a container body 110 configured to contain a liquid medicine, and an outlet port 120 attached to container body 110. Fig. 5 is a cross-sectional view showing a shape of the container body included in the liquid medicine container of the applicator according to an embodiment of the present invention. Fig. 6 is a cross-sectional view showing a shape of the outlet port included in the liquid medicine container of the applicator according to an embodiment of the present invention.

As shown in Figs. 2, 3, and 5, container body 110 is in a shape of a bottomed cylinder, configured to contain a liquid medicine in a receptacle 111, and has a top end portion 112 that is opened. An outer periphery of top end portion 112 has an external thread 113 to be engaged with an internal thread on an inner periphery of cap 180.

As shown in Figs. 2, 3, and 6, outlet port 120 includes a flange 121 having an annular external shape, a first tubular portion 122 protruding from flange 121 and having a cylindrical external shape, and a second tubular portion 123 protruding from flange 121 in the direction opposite to first tubular portion 122 and having a cylindrical external shape.

An opening 124 with diameter D1 is formed in a central portion of flange 121. Flange 121 has a portion surrounded by second tubular portion 123, and a top surface 125 of this portion is substantially flat. In the present embodiment, the external shape of flange 121, first tubular portion 122, second tubular portion 123, and opening 124 are located coaxially. The outer peripheral surface of first tubular portion 122 fits in the inner peripheral surface of top end portion 112 of container body 110. Accordingly, outlet port 120 and container body 110 are integrated into liquid medicine container 130.

As shown in Figs. 2, 3, and 6, brush member 160 is arranged adjacently to liquid medicine container 130 to cover opening 124, and brush member 160 is made up of a plurality of fibers 140 that are bundled together. Respective materials of a plurality of fibers 140 are not limited to the same materials, and may be different from each other. In the present embodiment, a plurality of fibers 140 are synthetic fibers. Respective shapes of a plurality of fibers 140 are not limited to the same shapes, and may be different from each other.

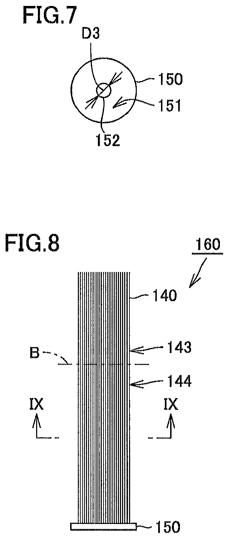

In the present embodiment, brush member 160 further includes an annular member 150 having one main surface to which a plurality of fibers 140 are bonded. Fig. 7 is a plan view showing an external appearance of the annular member included in the brush member of the applicator according to an embodiment of the present invention. As shown in Fig. 7, an opening 152 with diameter D3 is formed in a central portion of annular member 150. As shown in Figs. 2 and 3, D3 < D1 may hold where D1 = 4.0 mm and D3 = 2.0 mm, for example. Alternatively, D3 ≥ D1 may hold. A plurality of fibers 140 are bonded to one main surface 151 of annular member 150 with an adhesive.

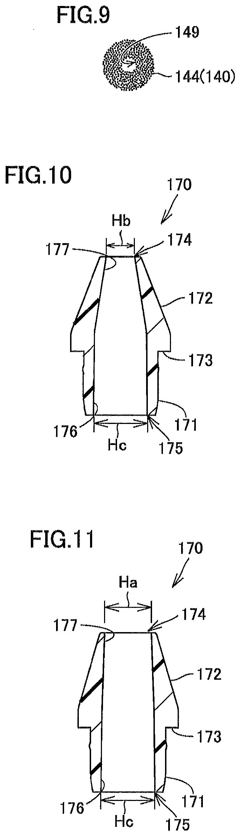

Fig. 8 is a side view showing an external appearance of the brush member of the applicator according to an embodiment of the present invention. In Fig. 8, the position of the topmost end of one end portion 174 of tubular member 170 described later herein is indicated by a dot-dash line B. Fig. 9 is a cross-sectional view of the brush member in Fig. 8 along line IX-IX as seen in the direction of arrows. As shown in Figs. 8 and 9, brush member 160 includes a root portion 144 in a cylindrical shape, and an opening 149 formed in a central portion of the root portion.

Fig. 10 is a cross-sectional view of the same cross section as Fig. 2, showing a shape of the tubular member of the applicator according to an embodiment of the present invention. Fig. 11 is a cross-sectional view of the same cross section as Fig. 3, showing a shape of the tubular member of the applicator according to an embodiment of the present invention.

As shown in Figs. 10 and 11, tubular member 170 includes a cylindrical portion 171, a hollow frustum portion 172, and a shoulder portion 173 located at the boundary between cylindrical portion 171 and hollow frustum portion 172. One end portion 174 located to extend from the topmost end of hollow frustum portion 172 has an inner peripheral surface 177 in an elliptical shape. Inner peripheral surface 177 has the major axis with its dimension represented by Ha, and the minor axis with its dimension represented by Hb. The other end portion 175 located to extend from the bottommost end of cylindrical portion 171 has an inner peripheral surface 176 in a circular shape. Inner peripheral surface 176 has the diameter with its dimension represented by He that is slightly larger than dimension Ha of the major axis of inner peripheral surface 177.

As shown in Figs. 2 and 3, the outer peripheral surface of cylindrical portion 171 fits in the inner peripheral surface of second tubular portion 123 of outlet port 120, and shoulder portion 173 is in contact with the topmost end of second tubular portion 123. Accordingly, tubular member 170 is fixed to liquid medicine container 130.

Annular member 150 is arranged adjacently to liquid medicine container 130 to allow an inner space of annular member 150 to continue into an inner space of opening 124. Specifically, annular member 150 is arranged adjacently to top surface 125 of flange 121, so as to allow opening 152 of annular member 150 to overlap opening 124 of liquid medicine container 130. The outer periphery of annular member 150 is located in a gap between top surface 125 of flange 121 and the other end portion 175 of second tubular portion 123.

As shown in Figs. 2 to 4, in tubular member 170, brush member 160 is inserted from the top end portion. A part 143 of the top end portion of brush member 160 is exposed to an outside of tubular member 170 from one end portion 174 of tubular member 170, where one end portion 174 is located opposite to liquid medicine container 130. Inner peripheral surface 177 of tubular member 170 is in contact with the outer periphery of brush member 160.

As shown in Figs. 2 to 4 and 10, brush member 160 has a tubular shape in a root portion 144 located adjacently to liquid medicine container 130, brush member 160 tapers from root portion 144 toward the top end portion located opposite to liquid medicine container 130, and brush member 160 has a wedge shape in the top end portion.

As shown in Fig. 4, dimension La of the length of a part 143 of the top end portion of brush member 160 is larger than dimension Ha of the width of the root of a part 143 of the top end portion of brush member 160. Dimension Ha of the width of the root of a part 143 of the top end portion of brush member 160 is larger than dimension Hb of the thickness of the root of a part 143 of the top end portion of brush member 160. Specifically, for example, La = 15 mm, Ha = 10 mm, and Hb = 5 mm.

Regarding applicator 100 in the present embodiment, brush member 160 has a tubular shape in its root portion 144, tapers from root portion 144 toward the top end portion, and the top end portion has a wedge shape. Therefore, the root portion of brush member 160 has appropriate stiffness, while the top end portion of brush member 160 has appropriate flexibility. Accordingly, the top end portion of brush member 160 is allowed to reach a site located deep in a narrow gap, and the top end portion of brush member 160 arriving at a deep site in the narrow gap can be flexed easily.

A liquid medicine delivered from opening 124 of liquid medicine container 130 passes through opening 152 of annular member 150 into opening 149 of brush member 160, and then passes through a part 143 of the top end portion of brush member 160 to be applied to a site for application. Thus, applicator 100 in the present embodiment can deliver the liquid medicine to a site located deep in a narrow gap while alleviating stimulus that may be given when the liquid medicine is applied.

Annular member 150 may not necessarily be arranged, and respective bottom ends of a plurality of fibers 140 may only be bonded together with an adhesive. If brush member 160 can keep the above-described shape without relying on tubular member 170, tubular member 170 may not be arranged. In this case, brush member 160 is bonded to outlet port 120.

Respective lengths of a plurality of fibers 140 are identical to each other. Therefore, brush member 160 is easy to manufacture, and the manufacturing cost of applicator 100 can be reduced.

### First Experimental Example

A first experimental example in which the top end portion of the brush member of the applicator is inserted in a narrow gap is now described. In the first experimental example, applicators of three types in total were prepared: the applicator disclosed in PTL 1 as Comparative Example 1, an applicator in the shape of a felt-tipped pen as Comparative Example 2, and the applicator in the present embodiment as Example 1.

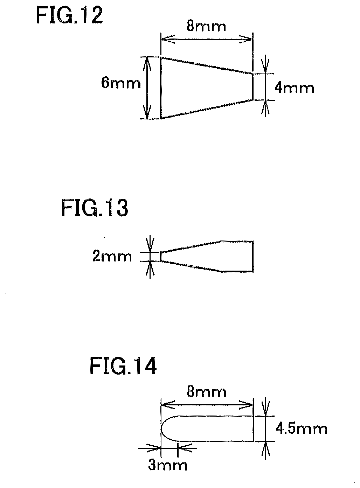

Fig. 12 is a plan view showing a shape of a part of a top end portion of the brush member of the applicator for Comparative Example 1. Fig. 13 is a side view showing a shape of a part of the top end portion of the brush member of the applicator for Comparative Example 1. Fig. 14 is a plan view showing a shape of a part of a top end portion of a brush member of the applicator for Comparative Example 2.

As shown in Figs. 12 and 13, the dimensions of a part of the top end portion of the brush member of the applicator for Comparative Example 1 were defined as follows: the length was 8 mm, the width of the root was 4 mm, the width of the topmost end was 6 mm, and the thickness of the topmost end was 2 mm. As shown in Fig. 14, the dimensions of a part of the top end portion of the brush member of the applicator for Comparative Example 2 were defined as follows: the length was 8 mm, the diameter of the root was 4.5 mm, and the length of a tapered portion was 3 mm.

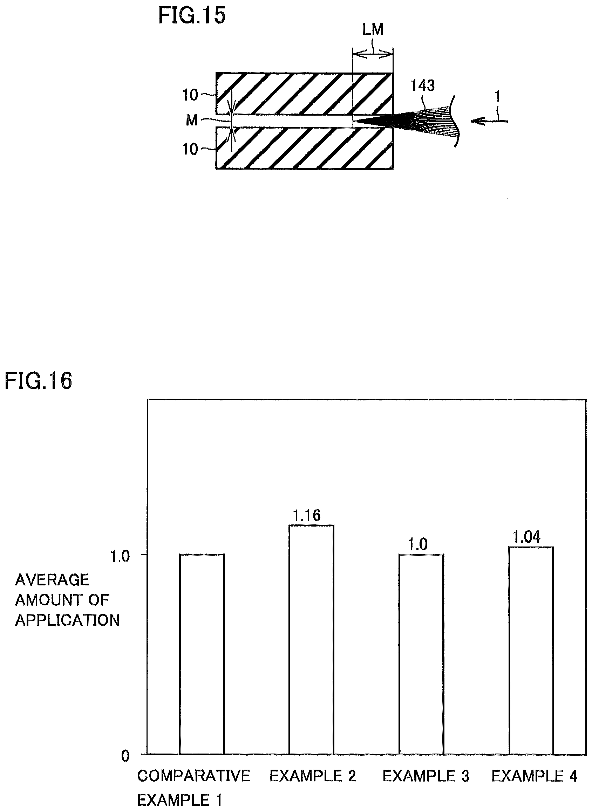

As to Example 1, the dimensions of a part 143 of the top end portion of brush member 160 were defined as follows: La = 10 mm, Hb = 3.5 mm. Fig. 15 is a cross-sectional view showing a state in which the top end portion of the brush member of the applicator for Example 1 is inserted in a narrow gap.

As shown in Fig. 15, in the first experimental example, a part of the top end portion of the brush member was applied, in the direction of arrow 1, into a gap M between two glass plates 10, and the inserted length LM of the brush member was measured. Four different gaps M: 0.15 mm, 0.2 mm, 0.5 mm, and 1.0 mm were prepared.

**[Table 1]**

| | M = 0.15 mm | M = 0.2mm | M = 0.5 mm | M = 1.0 mm |
|---|---|---|---|---|
| Comparative Example 1 | 0 mm | 0.5 mm | 2.0 mm | 5.0 mm |
| Comparative Example 2 | 0 mm | 0 mm | 1.0 mm | 1.5 mm |
| Example 1 | 5.0 mm | 5.0 mm | 6.0 mm | 6.5 mm |

The results of the first experimental example are indicated in Table 1. As shown in Table 1, respective inserted lengths LM in gap M = 0.15 mm were: 0 mm for Comparative Example 1, 0 mm for Comparative Example 2, and 5.0 mm for Example 1. Respective inserted lengths LM in gap M = 0.2 mm were: 0.5 mm for Comparative Example 1, 0 mm for Comparative Example 2, and 5.0 mm for Example 1, Respective inserted lengths LM in gap M = 0.5 mm were: 2.0 mm for Comparative Example 1, 1.0 mm for Comparative Example 2, and 6.0 mm for Example 1. Respective inserted lengths LM in gap M = 1.0 mm were: 5.0 mm for Comparative Example 1, 1.5 mm for Comparative Example 2, and 6.5 mm for Example 1.

It was confirmed from the results of the first experimental example that applicator 100 of Example 1 was capable of inserting the top end portion of brush member 160 to a site located deep in a narrow gap, as compared with respective applicators of Comparative Examples 1 and 2.

### Second Experimental Example

Next, a second experimental example is described in which a relation between diameter D3 of opening 152 of annular member 150 and the amount of application of a liquid medicine was examined. In the second experimental example, applicators of four types in total were prepared: the applicator disclosed in PTL 1 as Comparative Example 1, as well as applicators of three types in the present embodiment that were specifically an applicator with diameter D3 of 2.1 mm as Example 2, an applicator with diameter D3 of 2.0 mm as Example 3, and an applicator with diameter D3 of 1.8 mm as Example 4.

For the applicators of the four types, the amount of application was measured by measuring the weight of the applicator after application to a site for 30 seconds. The liquid was applied ten times, and the average of respective amounts of application performed ten times was determined. The determined average amount of application was standardized with respect to an average amount of application of 1.0 from the applicator of the Comparative Example.

Fig. 16 is a graph showing the results of the second experimental example. As shown in Fig. 16, the average amount of application of Example 2 was 1.16, that of Example 3 was 1.0, and that of Example 4 was 1.04. It was confirmed that any of these Examples can provide an amount of application substantially equivalent to the Comparative Example.

It was confirmed, from the results of the first and second experimental examples, that the applicator in the present embodiment was capable of applying the liquid medicine to a site located deep in a narrow gap.

The embodiments and the examples disclosed herein are given by way of illustration in all respects, not for serving as a basis for restrictive interpretation. The technical scope of the present invention is therefore defined based on claims, rather than based solely on the embodiments and the examples described above, and encompasses all modifications and variations equivalent in meaning and scope to the claims.

### REFERENCE SIGNS LIST

1 liquid drug; 10 glass plate; 100 applicator; 110 container body; 111 receptacle; 112 top end portion; 113 external thread; 120 outlet port; 121 flange; 122 first tubular portion; 123 second tubular portion; 124, 152 opening; 125 top surface; 130 liquid medicine container; 140 fiber; 143 part of top end portion; 144 root portion; 149 opening; 150 annular member; 151 main surface; 160 brush member; 170 tubular member; 171 cylindrical portion; 172 hollow frustum portion; 173 shoulder portion; 174 one end portion; 175 the other end portion; 176, 177 inner peripheral surface; 180 cap

## Claims

1. An applicator comprising:
a liquid medicine container having an opening; and
a brush member arranged adjacently to the liquid medicine container to cover the opening, the brush member being made up of a plurality of fibers that are bundled together,
the brush member
having a tubular shape in a root portion located adjacently to the liquid medicine container,
tapering from the root portion toward a top end portion located opposite to the liquid medicine container, and
having a wedge shape in the top end portion.

2. The applicator according to claim 1, further comprising
a tubular member in which the brush member is inserted from the top end portion, and which is secured to the liquid medicine container, wherein
a part of the top end portion of the brush member is exposed to an outside of the tubular member from one end portion, located opposite to the liquid medicine container, of the tubular member,
the one end portion has an inner peripheral surface in an elliptical shape, and the inner peripheral surface is in contact with an outer periphery of the brush member.

3. The applicator according to claim 1 or 2, wherein
the brush member further includes an annular member having one main surface to which the plurality of fibers are bonded, and
the annular member is arranged adjacently to the liquid medicine container to allow an inner space of the annular member to continue into an inner space of the opening.

4. The applicator according to claim 2 or 3, wherein a dimension of a length of the part of the top end portion of the brush member is larger than a dimension of a width of a root of the part of the top end portion of the brush member.

5. The applicator according to any one of claims 1 to 4, wherein respective lengths of the plurality of fibers are identical to each other.

6. A liquid drug applicator comprising:
an applicator according to any one of claims 1 to 5; and
a liquid drug contained in the liquid medicine container.
